# EUROPEAN PATENT APPLICATION

(11) **EP 4 386 011 A1**
(43) Date of publication of application: **19.06.2024**
(21) Application number: 22855544.7
(22) Date of filing: 12.08.2022
(51) Int. Cl.: C07K 19/00, C12N 15/62, C12N 15/85, C12N 5/10, A61K 47/68, A61K 38/21, A61P 35/00

(54) **GPC3-TARGETED ANTIBODY INTERFERON ALPHA FUSION PROTEIN AND USE THEREOF**

(30) Priority: 12.08.2021 CN 202110926743
(71) Applicant: Shanghai JMT-Bio Technology Co., Ltd., Shanghai 200032 (CN)
(72) Inventor: HE, Ke, Shanghai 201318 (CN); SONG, Liping, Shanghai 201318 (CN); FAN, Yi, Shanghai 201318 (CN); CHEN, Yingjiao, Shanghai 201318 (CN)
(74) Representative: Longland, Emma Louise
(86) International application number: PCT/CN2022/112278
(87) International publication number: WO 2023/016564

(57) **Abstract**

A GPC3-targeting antibody-interferon α fusion protein and a use thereof are disclosed in this invention. The fusion protein consists of an A chain and a B chain; wherein the amino acid sequence of the A chain is as shown in SEQ ID NO: 1, and the amino acid sequence of the B chain is as shown in SEQ ID NO: 2. The killing effect on target cells, especially tumor cells, can be significantly improved, and toxic side effects caused by binding to non-target cells are significantly reduced. The fusion protein has better safety, lower production cost, and good freeze-thaw stability.

## Description

This application claims priority from Chinese patent application 2021109267431 with a filing date of 2021/8/12.

### Technical field

The invention belongs to the field of biomedicine, and specifically relates to a GPC3-targeting antibody-interferon α fusion protein and a use thereof

### Background

Interferon α (IFNα) has a wide range of antiviral and anti-tumor effects. It activates effector cells through binding to IFNα receptors, increases the activities of immune cells such as natural killer cells, inhibits the proliferation of viruses and tumor cells, and induces tumor cell apoptosis, etc.. Therefore, it is one of the biological products widely used in clinical practice. It is mainly used in the treatment of liver diseases (such as chronic hepatitis B, hepatitis C, etc.), antiviral uses (such as viral respiratory diseases, skin diseases, blood diseases, gynecological diseases, etc.) and specific tumors (such as chronic myeloid leukemia, melanoma, lymphoma, renal cancer, etc.), and is used as an adjuvant therapy for tumors and hematological malignancies.

However, since IFNα receptors are widely expressed in various tissues and organs of human body, it is often accompanied by toxic side effects such as fever, fatigue, myalgia, liver toxicity, bone marrow suppression, and neurotoxicity during clinical use. A large amount of clinical research data shows that PEG-IFNα (6 µg/kg/week for 8 weeks of treatment; 3 µg/kg/week for maintenance) will cause more than 60% of patients to have serious adverse events (SAE) of grade 3 or above during the adjuvant treatment of melanoma (Practical Guidelines for the Management of Interferon-a-2b Side Effects in Patients Receiving Adjuvant Treatment for Melanoma. Cancer 2008; 112:982-94). The clinical maximum tolerance dose (MTD) of PEG-IFNα in the treatment of chronic myeloid leukemia CML is 7.5-9µg/kg (Long-Term Results of the Randomized Phase III Trial EORTC 18991 of Adjuvant Therapy With Pegylated Interferon Alfa-2b Versus Observation in Resected Stage III Melanoma. JOURNAL OF CLINICAL ONCOLOGY, VOLUME 30, NUMBER 31, NOVEMBER 1 2012). According to preclinical in vitro and in vivo drug efficacy experimental data, the optimal tumor inhibitory dose needs to be at least 100 times higher than the clinically tolerance dose (Treating Cancer with PEG Intron Pharmacokinetic Profile and Dosing Guidelines for an Improved Interferon-A-2b Formulation. TALPAZ et al BLOOD, 15 SEPTEMBER 2001. VOLUME 98, NUMBER 6).

In addition, interferon α generally has poor freeze-thaw stability due to its product characteristics. For example, the aggregates of recombinant human albumin interferon-α2b fusion protein (rHSA-IFN-α2b) gradually increase after repeated freezing and thawing. After freezing and thawing four times, the content of aggregates reaches 17.91%. The recombinant human albumin interferon-α2b fusion protein is not suitable for freezing and thawing (Xia Yi et al., Study on stability of recombinant human albumin interferon α-2b fusion protein, Journal of Biology, 2008, 25(006):38-40), and results in more restrictions on the production, transportation and use.

Glypican 3 (GPC3) is a member of the glypican family of heparan sulfate proteoglycans present on the surface of cells. Existing experimental studies have shown that GPC3 mRNA and GPC3 protein are specifically highly expressed in liver cancer and are closely related to the occurrence and development of liver cancer [Zhu ZW, Friess H, WangL, Abou-Shady M, Zimmermann A, LanderAD, KorcM, KleeffJ, Büchler MW.Gut 2001; 48: 558-64]. Existing studies and literature reports have shown that GPC3 is closely related to the occurrence and development of liver cancer. Not only does it have a higher detection rate in the early stages of liver cancer, but its detection rate also increases as liver cancer progresses. In addition, studies have found that GPC3 expression ratio is higher in lung squamous cell carcinoma (LSCC), ovarian cancer, and yolk sac tumor.

GPC3 has obvious sensitivity and specificity in the diagnosis of liver cancer and can be used as a new target for liver cancer treatment. Codrituzumab (GC33) is the first antibody targeting GPC3 and is currently in clinical phase II. However, preliminary results of its clinical studies show that GC33 does not show clinical benefit in the HCC population. It can be seen that the effect of monoclonal antibody drug GC33 targeting GPC3 in clinical phase II is poor, and there is no significant difference in the overall survival rate and progression-free survival rate between the drug group and the control group.

In addition, the anti-GPC3 monoclonal antibody Codrituzumab (GC33) and IFN-α 2b are extremely incompatible in terms of clinical medication mode, dosage, half-life and other aspects. For example, IFN-α 2b is generally injected subcutaneously, and the human body's tolerance dose is about 20ug/kg, with a half-life of only 3-4 hours; while the anti-GPC3 monoclonal antibody Codrituzumab (GC33) is injected intravenously, and the maximum tolerance dose in clinical Phase I can reach 20mg/kg per week, but it has not yet achieved optimal efficacy, and its half-life exceeds 4 days. Therefore, to simultaneously achieve better tumor treatment effects than IFN-α2b and the anti-GPC3 monoclonal antibody Codrituzumab (GC33) and reduce the toxic side effects of IFN-α 2b, a simple combination or fusion cannot achieve compatibility.

### Summary of the invention

The technical problem to be solved by the present invention is to overcome the poor efficacy of anti-GPC3 monoclonal antibodies (such as GC33) and the deficiencies in the safety and freeze-thaw stability of interferon α in the prior art, and provide an antibody-interferon α fusion protein targeting GPC3 and the use thereof. The antibody-interferon α fusion protein can significantly improve the targeting ability of the interferon α functional fragment in the fusion protein and improve the killing effect of the fusion protein on target cells (such as GPC3-positive tumor cells) while reduce the toxic side effects of the interferon α functional fragment on normal cells (healthy cells that do not express GPC3). At the same time, it significantly reduces the toxic side effects of potential impurity molecules containing the interferon α functional fragment generated during the preparation process on non-target organs, tissues, and cells.

The present invention solves the above technical problems by the following technical solutions.

The first aspect of the present invention provides an antibody-interferon α fusion protein targeting GPC3, which consists of an A chain and a B chain; wherein the amino acid sequence of the A chain is as shown in SEQ ID NO: 1, and the amino acid sequence of the B chain is as shown in SEQ ID NO:2.

The second aspect of the invention provides an isolated nucleic acid encoding the antibody-interferon α fusion protein as described in the first aspect.

The third aspect of the present invention provides a recombinant expression vector comprising the nucleic acid as described in the second aspect.

In the present invention, the recombinant expression vector is formed by introducing an exogenous nucleic acid into a vector.

The fourth aspect of the present invention provides a transformant comprising the recombinant expression vector as described in the third aspect.

In the present invention, the transformant is formed by introducing the recombinant expression vector into a host cell.

The fifth aspect of the present invention provides a method for preparing an antibody-interferon α fusion protein targeting GPC3. The method comprises the following steps: culturing the transformant as described in the fourth aspect, and obtaining the antibody-interferon α fusion protein from the culture.

The sixth aspect of the present invention provides a pharmaceutical composition, which comprises the antibody-interferon α fusion protein as described in the first aspect and a pharmaceutically acceptable carrier.

The seventh aspect of the present invention provides use of the antibody-interferon α fusion protein as described in the first aspect or the pharmaceutical composition as described in the sixth aspect in the manufacture of a medicament for the treatment, auxiliary treatment or prevention of a disease.

In some embodiments of the invention, the disease is a GPC3-positive related disease.

In the present invention, the GPC3-positive related disease is selected from a tumor, a liver disease and a viral infection disease.

In some embodiments of the present invention, the tumor is selected from breast cancer, intestinal cancer, pancreatic cancer, esophageal cancer, ovarian cancer, gastric cancer, prostate cancer, kidney cancer, cervical cancer, myeloma, lymphoma, leukemia, thyroid cancer, uterine cancer, bladder cancer, neuroendocrine cancer, head and neck cancer, liver cancer, nasopharyngeal cancer, testicular cancer, lung cancer, melanoma, skin cancer, sarcoma, glioma, mesothelioma and myelodysplastic syndrome.

In the present invention, the intestinal cancer comprises colorectal cancer, the ovarian cancer comprises yolk sac tumor, the neuroendocrine cancer comprises Merkel cell carcinoma, the lung cancer comprises small cell lung cancer and non-small cell lung cancer, the skin cancer comprises basal cell skin cancer and squamous cell skin cancer, the sarcoma comprises dermatofibrosarcoma protuberans, the gliomas comprises glioblastoma, and the uterine cancer comprises endometrial cancer and uterine sarcoma.

The eighth aspect of the present invention provides a method for treating a GPC3-positive related disease, which comprises administering or administering in combination with a therapeutically effective amount of the antibody-interferon α fusion protein as described in the first aspect or the pharmaceutical composition described in the sixth aspect to a patient in need thereof.

As used herein, the term "recombinant protein" refers to an artificially designed/constructed protein rather than a naturally occurring protein. The word "recombinant" in the "recombinant protein" of the present invention does not represent its production method, it is only used to indicate that the "recombinant protein" does not exist naturally. The recombinant protein of the present invention can be an expressed protein or an assembled protein.

As used herein, the term "fusion protein" refers to an expression product obtained by recombining two genes through DNA recombinant technology. Fusion protein technology can be used to construct and express new target proteins with multiple functions.

As used herein, the term "antibody" generally refers to a protein comprising one or more polypeptides substantially encoded by an immunoglobulin gene or an immunoglobulin gene fragment. Immunoglobulin genes can comprise κ, λ, α, γ, δ, ε and µ constant region genes, as well as numerous immunoglobulin variable region genes. As used herein, light chains may be classified as κ or λ. Heavy chains can be classified as γ, µ, α, δ or ε, which in turn define the immunoglobulin classes: IgG, IgM, IgA, IgD and IgE respectively. Antibodies used in the present application may have structural units comprising tetramers. Each tetramer can be composed of two pairs of identical polypeptide chains, each pair having a "light" chain (approximately 25 kD) and a "heavy" chain (approximately 50-70 kD). The N-terminus of each structural unit may define a variable region of approximately 100 to 110 or more amino acids, which is primarily responsible for antigen recognition. As used herein, the terms light chain variable region (VL) and heavy chain variable region (VH) generally refer to these regions of the light chain and heavy chain, respectively. Antibodies may exist as intact immunoglobulins or as a number of well-characterized fragments generated by digestion with various peptidases or by de novo expression (for more detailed description of other antibody fragments, see Fundamental Immunology, edited by W.E. Paul, Raven Press, N.Y. (1993)).

As used herein, the term "Fc region" (fragment crystallizable, Fc) consists of IgG constant regions CH2 and CH3 domains and the hinge region.

As used herein, the term "antigen-binding fragment" or "Fab fragment" or "Fab" consists of the variable region of the light chain (VL), the constant region of the light chain (CL), the variable region of the heavy chain (VH), and the constant region 1 of the heavy chain (CH1) domain, which can bind to antigen.

As used herein, the term "Knob(s)-into-Hole(s) technology" or "pestle and mortar" technology or "button" technology uses genetic engineering technology to introduce different mutations into the two CH3 domains of the heavy chains to promote heterodimerization of the heavy chains. A knob is made on one heavy chain and a hole is made on the other heavy chain, and then the two are preferentially snapped together to form an asymmetric antibody (Ridgway JB, et al.'Knobs-into-holes'engineering ofantibody CH3domains for heavy chain heterodimerization. Protein Engineering,1996,9(7):617-621). As known by those skilled in the art, multiple knobs and/or holes can be made on one heavy chain, and correspondingly, multiple holes and/or knobs can be made on the other heavy chain.

As used herein, the term "antigen specificity" refers to a specific antigen or epitope thereof that is selectively recognized by an antigen-binding molecule.

As used herein, the term "substitution" when applied to amino acid residues refers to the substitution of one or more naturally occurring or introduced amino acids in a peptide, polypeptide or protein with another one to form a new peptide, polypeptide or protein (such as the term "mutant" or "mutantion" herein). Substitutions in a polypeptide or protein can result in reduced or unchanged function of the polypeptide or protein. Substitutions can also be "conservative substitutions", which in the context of an amino acid sequence refers to the replacement of one amino acid residue with a different amino acid residue having a side chain that has similar physicochemical properties, or substitutions of amino acids which are not critical to the activity of the polypeptide. For example, conservative substitutions can be made between non-polar side chain amino acid residues (such as Met, Ala, Val, Leu and Ile, Pro, Phe, Trp), between uncharged polar side chain residues (such as Cys, Ser, Thr, Asn, Gly and Gln), between acidic side chain residues (such as Asp, Glu), between basic side chain amino acids (such as His, Lys and Arg), between β-branched side chain amino acids (such as Thr, Val and Ile), between sulfur-containing side chain amino acids (such as Cys and Met) or between aromatic side chain residues (such as Trp, Tyr, His and Phe). In certain embodiments, substitutions, deletions, or additions may also be considered as "conservative substitutions". The number of inserted or deleted amino acids may range from about 1 to 5. Conservative substitutions usually do not cause significant change the conformation and structure of a protein, and therefore maintain the biological activity of the protein.

As used herein, the term "double-positively expressing cell" or "target cell" or "target cell of interest" refers to a cell that can simultaneously bind to a GC33 antigen-binding fragment and interferon α functional fragment.

As used herein, the term "non-target cells" or "non-target cell of interest" refers to a cell that does not express GPC3 and only interacts with interferon α functional fragment.

As used herein, the term "IFNα" or "α type interferon" or "interferon α" includes all natural or recombinant α interferons, particularly preferably human α interferons, such as recombinant human α interferons, including but not limited to, IFN-α2b (for example, Intron^{®}A interferon or Viraferon^{®}-A interferon available from Schering Corporation, Kenilworth, N.J.), IFN-α 2a (for example, Roferon^{®}-A interferon available from Hoffmann-La Roche, Nutley, N.J.); such as a mixture of natural α interferons, including but not limited to IFN-α-n1 (for example, Wellferon^{®} interferon α-n1 available from Sumitomo, Japan or Glaxo-Wellcome Ltd., London, Great Britain) or IFN-α-n3 (for example, Alferon N^{®} interferon available from Interferon Sciences). In the present invention, the term "IFNα" or "α type interferon" or "interferon α" or "interferon α functional fragment" or "IFNα functional fragment" also includes any substance with IFNα biological activity, such as mutated or truncated or modified IFNα, such as IFNα low-affinity mutants, PEG derivatives of IFNα (PEG-IFNα). In the present invention, the term "IFNα" or "α type interferon" or "interferon α" is not limited to any specific source of acquisition and can be obtained from commercial sources or produced by conventional techniques known to those skilled in the art. The production method includes but is not limited to biological source extraction method and genetic engineering extraction method, which are described in detail in, for example, "Pestka S. Arch Biochem Biophys. 1983 Feb 15; 221 (1): 1-37". In some embodiments, IFNα is from a species selected from the group consisting of human, equine, bovine, murine, porcine, rabbit, cat, dog, rat, goat, sheep, and non-human primate.

As used herein, the term "IFNα2b" or "IFN-α2b" or "IFN-α 2b" or "interferon-α 2b" is a subtype of IFN-α and is an expression of interferon-α 2b. The amino acid sequence of human derived wild-type interferon-α 2b containing a signal peptide is shown in SEQ ID NO: 3.

As used herein, the term "low affinity mutant" refers to an IFNα mutant which has an unchanged or reduced proliferation inhibition or antiviral functional activity compared to wild-type IFNα.

As used herein, the term "linker sequence" or "Linker" refers to an amino acid sequence which ligating different functional binding fragments (such as GC33 antigen-binding fragment and interferon α functional fragment, GC33 antigen-binding fragment or interferon α functional fragment and Fc region), or ligating different domains within the same functional binding fragment.

As used herein, the terms "GC33", "Codrituzumab", "Anti-GPC3 mAb", "anti-GPC3 Ab" are used interchangeably in the present invention, and refer to the anti-GPC3 antibody Codrituzumab.

As used herein, the term "recombinant protein of the invention" refers to an antibody-interferon α fusion protein targeting GPC3, specifically refers to TTM101-LC03-M3.

The term "host cell" as used herein generally includes a single cell, a cell line or a cell culture that can be or has been the recipient of a plasmid or vector of a subject, which comprises a polynucleotide disclosed herein, or which expresses the protein heterodimers (e.g., heterodimeric proteins) of the present invention. A host cell can include progeny of a single host cell. Due to natural, accidental or intentional mutations, the offspring may not necessarily be identical to the original parent cell (either morphologically or in terms of total genomic DNA complement). Host cells may include cells transfected in vitro with vectors disclosed herein. The host cells may be bacterial cells (e.g, E. coli), yeast cells, or other eukaryotic cells, such as HEK293 cells, COS cells, Chinese Hamster Ovary (CHO) cells, HeLa cells, or myeloma cells. In some embodiments, the host cells are a mammalian cells. In some embodiments, the mammalian cells are CHO cells.

As used herein, the term "vector" generally refers to a nucleic acid molecule capable of self-replication in a suitable host, which transfers an inserted nucleic acid molecule into and/or between host cells. The term may include vectors used primarily for the insertion of DNAs or RNAs into a cells, vectors used primarily for the replication of DNAs or RNAs, and expression vectors used for the transcription and/or translation of DNAs or RNAs. Also included are vectors that provide more than one of the above functions. An "expression vector" is a polynucleotide that can be transcribed and translated into a polypeptide when introduced into a suitable host cell. An "expression system" generally means a suitable host cell containing an expression vector capable of producing desired expression yield.

The term "effective amount" or "therapeutically effective amount" refers to an amount of a composition (e.g., a recombinant protein described herein) sufficient to achieve the intended use (including but not limited to treatment of diseases). The therapeutically effective amount may vary depending on the intended use (e.g., in vitro or in vivo) or the subject and disease condition being treated, such as the weight and age of the subject, the severity of the disease condition, the mode of administration, etc., which may be readily be determined by those of ordinary skill in the art. The term may also be applied to a dosage that induces a specific response in a target cell (e.g., target gene induction, proliferation, and/or apoptosis). The specific dosage will vary depending on the particular compound selected, the dosing regimen followed, whether it is administered in combination with other compounds, the timing of administration, the tissue to which it is administered, and the physical delivery system in which it is administered.

The terms "treatment" or "treating" or "mitigation" or "amelioration" are used interchangeably herein and refer to methods of obtaining beneficial or desired results (including, but not limited to, therapeutic benefits and/or preventive benefits). As used herein, therapeutic benefits generally refer to eradication or reduction of the severity of the underlying condition being treated. Additionally, by eradicating, reducing the severity, or reducing the incidence of one or more physical symptoms associated with the underlying condition such that improvement is observed in the subject (although the subject may still be suffering from the underlying condition), therapeutic benefits can be achieved. For prophylactic benefits, the composition may be administered to a subject who is at risk of developing a particular disease, or who reports one or more physiological symptoms of a disease, even though a diagnosis of the disease may not have been made.

As used herein, the terms "co-administering," "administering in combination with," and their grammatical equivalents generally include the administration of two or more agents to an animal such that the agents and/or their metabolites are simultaneously present in the subject. Co-administration includes simultaneous administration in separate compositions, administration at different times in separate compositions, or administration in a composition in which both agents are present.

As used herein, the term "cell proliferation" generally refers to the phenomenon of a change in cell number due to division. For example, cell proliferation can result in an increase in cell number. The term also includes cell growth by which the cell morphology has been altered (e.g., increased in size), which is consistent with a proliferative signal.

As used herein, the term "proliferation inhibition" or "inhibition of cell proliferation" generally refers to a reduction in the growth rate and/or proliferation rate of cancer cells. For example, this may include death of cancer cells (e.g., by apoptosis). In some embodiments, the term may also refer to inhibiting the growth and/or proliferation of solid tumors and/or inducing size reduction or elimination of tumors.

As used herein, the term "freeze-thaw stability" generally refers to the stability of an emulsion system when subjected to alternating freezing and thawing.

[ The term "subject" or "individual" or "animal" or "patient" as used herein refers to human or non-human animals, including mammals or primates. Mammalian subjects include human, livestock animals, farm animals, and zoo, sporting or pet animals, such as dogs, cats, guinea pigs, rabbits, rats, mice, horses, pigs, cows, bears, etc.

As used herein, the term "in vivo" generally refers to events that occur within the body of a subject.

As used herein, the term "in vitro" generally refers to events that occur outside the body of a subject. For example, in vitro assays include any assay performed outside a subject. In vitro assays include cell-based assays in which dead or live cells are used.

In vitro assays also include cell-free assays in which intact cells are not used.

As used herein, the term "administration" refers to delivering to a subject a therapeutically effective amount of a pharmaceutical composition comprising a recombinant protein or fusion protein of the invention. Administration may be systemic or topical. Administration can be made by means of an administration device, such as a syringe. Methods of administration include but are not limited to encapsulation, nasal inhalation, spraying, injection, etc. Routes of administration include inhalation, intranasal, oral, intravenous, subcutaneous or intramuscular administration, etc.

Compared to the prior art, the present invention has the following beneficial effects:
The new GPC3-targeting antibody-interferon α fusion protein of the present invention has extremely strong proliferation inhibition and tumor killing activity on GPC3-positive target cells (tumor cells, such as liver cancer cells HepG2 and HuH-7), while is extremely safe to non-target cells which do not express GPC3. In addition, the freeze-thaw stability of the fusion protein is significantly better than that of interferon α, which is beneficial to the production, transportation and use of the product.

For cells expressing IFNα receptors, the beneficial effects are as follows:
1. The binding activity, ADCC activity, proliferation inhibitory activity, etc. of the recombinant protein of the present invention on GPC3-positive target cells (such as liver cancer cells HepG2 and HuH-7) are significantly stronger than that of IFNα at equal molar concentrations, and its binding activity and proliferation inhibitory activity etc. on GPC3 negative non-target cells (such as normal cells) are unexpectedly weaker than IFNα at equal molar concentrations. In some experiments, the detection results of the proliferation inhibitory activity of the recombinant protein of the present invention on GPC3-negative non-target cells were negative. That is, when the non-target cells do not have the ability of binding to the GC33 antigen-binding fragment, the binding activity and proliferation inhibitory activity etc. of the IFNα functional fragment of the recombinant protein of the present invention on cells expressing IFNα receptors are significantly reduced or even no relevant activity is found.
2. The potential risk impurity (homodimer of B chains) of the recombinant protein of the present invention has extremely weak inhibitory activity on the proliferation of GPC3-negative non-target cells. Compared to the bispecific recombinant protein of CN108864290B, the potential risk impurity of the recombinant protein of the present invention has weaker inhibitory activity on the proliferation of GPC3-negative non-target cells. Therefore, the safety risks or toxic side effects caused by the potential risk impurity of the recombinant protein of the present invention are extremely low. At the same time, high production cost caused by quality control of risk impurity is reduced.
3. The recombinant protein of the present invention has good freeze-thaw stability. After repeated freezing and thawing of 5 times, the purity is above 95%, the appearance is clear, and the freeze-thaw stability is significantly better than that of IFN-α2 b monomer or PEGylated IFN- α2b.

To sum up, the recombinant protein of the present invention can significantly improve the killing effect on target cells, especially tumor cells, while significantly reduce the toxic side effects caused by binding to non-target cells, with better safety and lower production cost and good freeze-thaw stability.

### Brief description of drawings

Figure 1 is a schematic structural diagram of the recombinant protein of the present invention.
Figure 2 is a non-reducing SDS-PAGE electrophoresis diagram of the purified recombinant protein of the present invention.
Figure 3 is a graph of the binding activity of the recombinant protein of the present invention and the control sample on the target cell HuH-7 and the non-target cell MDA-MB-231 measured by flow cytometry.
Figure 4 is a graph of the ADCC activity of the recombinant protein of the present invention on the target cell HuH-7 measured by reporter gene method.
Figure 5 is a graph of the proliferation inhibitory activity of the recombinant protein of the present invention on the GPC3-positive target cell HuH-7.
Figure 6A is a graph of the proliferation inhibitory activity of the recombinant protein of the present invention on the GPC3-negative non-target cell MDA-MB-231.
Figure 6B is a graph of the proliferation inhibitory activity of the recombinant protein of the present invention on the GPC3-negative non-target cell U266.
Figure 7 is a bar chart of the proliferation inhibitory activity of the potential risk impurity of the recombinant protein of the present invention on the GPC3-negative non-target cell MDA-MB-231.
Figure 8 is a graph of the anti-tumor efficacy of the recombinant protein of the present invention on BALB/c nude mouse HuH-7 subcutaneous tumor model.
Figure 9 is a bar chart of the anti-tumor efficacy of the recombinant protein of the present invention on BALB/c nude mouse HuH-7 subcutaneous tumor model.

### Detailed description

In order to make it easy to understand the technical means, creative features, objectives and effects achieved by the invention, the invention is further elaborated below with reference to specific illustrations. However, the present invention is not limited to the following implementation examples.

The concepts, specific structures and technical effects of the present invention will be further described below in conjunction with the accompanying drawings, so as to fully explain the purposes, features and effects of the present invention.

### Example 1 Design of the recombinant protein

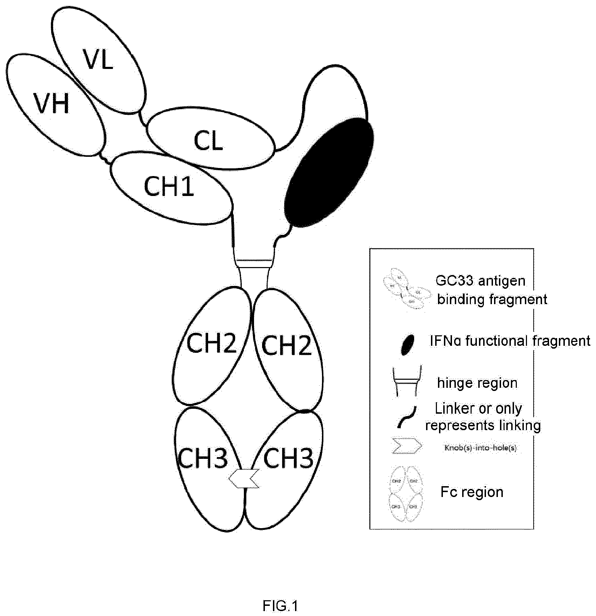

The structure of the recombinant protein of the present invention is shown in Figure 1. The recombinant protein comprises a GC33 antigen-binding fragment, an IFNα functional fragment and an Fc region, wherein the N-terminus of the IFNα functional fragment is ligated to the C-terminus of CL in the antigen-binding fragment through (GGGGS)₄ (as shown in Figure 1), the C-terminus of the IFNα functional fragment is directly ligated to the Fc region, and the variable region (V region) and the constant region (C region) in the antigen-binding fragment are directly ligated. The Fc region uses knobs-into-holes technology.

This example takes TTM101-LC03-M3 as an example to illustrate the design of the recombinant protein of the present invention. The molecular structure information of TTM101-LC03-M3 is shown in Table 1, and the control samples are shown in Table 2.

**Table 1 Exemplary molecular structure of the recombinant protein of the present invention**

| Number of the recombinant protein | The first functional antibody ×the second functional antibody | Exemplary composition of bispecific antibody molecule | |
|---|---|---|---|
| | | A chain | B chain |
| TTM101-LC03-M3 | Anti-GPC3 mAb × IFN-α 2b (A145G) | GC33(H)-Fc1(Hole) | GC33(L)-(GGGGS)₄-IFNα2b (A145G)-Fc2(Knob) |

**Table 2 Exemplary molecular structures of control samples**

| Name of the control sample | Composition of the molecule |
|---|---|
| GC33 | Anti-GPC3 monoclonal antibody codrituzumab |
| IFN-α 2b | Recombinantly expressed IFN-α 2b protein |

In the above Tables 1 and 2, (H) refers to the domain consisting of heavy chain VH and CH1, (L) refers to the domain consisting of light chain VL and CL; GC33 represents the anti-glypican3 (GPC3) monoclonal antibody codrituzumab; Fc represents the wild-type Fc region, Fc1 represents the Fc region with hole or holes mutation, and Fc2 represents the Fc region with knob or knobs mutation.

The sequence ID numbers corresponding to the sequence names mentioned in Table 1 are shown in Table 3. Among them, the signal peptide of A chain and B chain is shown in SEQ ID NO:8; the amino acid sequence of IFN-α 2b is shown in SEQ ID NO:3, wherein positions 1 to 23 are the signal peptide sequence; the amino acid sequence of Codrituzumab is cited from Patent US7919086, the heavy chain amino acid sequence is shown in SEQ ID NO:6, and the light chain amino acid sequence is shown in SEQ ID NO:7. Human IgG1 isotype control (B117901) was purchased from Biointron Biotech. Recombinantly expressed IFN-α2b protein (Z03003) was purchased from Nanjing GenScript Biotechnology Co., Ltd.

**Table 3 Sequence names and corresponding sequence ID numbers**

| Sequence name | SEQ ID NO | sequence |
|---|---|---|
| GC33(H)-Fc1 | 1 | QVQLVQSGAEVKKPGASVKVSCKASGYTFTDYEMHWVRQAPGQGLE WMGALDPKTGDTAYSQKFKGRVTLTADKSTSTAYMELSSLTSEDTAVY YCTRFYSYTYWGQGTLVTVSSASTKGPSVFPLAPSSKSTSGGTAALGCL VKDYFPEPVTVSWNSGALTSGVHTFPAVLQSSGLYSLSSVVTVPSSSLGT QTYICNVNHKPSNTKVDKKVEPKSCDKTHTCPPCPAPELLGGPSVFLFP PKPKDTLMISRTPEVTCVVVDVSHEDPEVKFNWYVDGVEVHNAKTKP REEQYNSTYRVVSVLTVLHQDWLNGKEYKCKVSNKALPAPIEKTISKA KGQPREPQVYTLPPSRDELTKNQVSLSCAVKGFYPSDIAVEWESNGQPE NNYKTTPPVLDSDGSFFLVSKLTVDKSRWQQGNVFSCSVMHEALHNH YTQKSLSLSPGK |
| GC33(L)-(GGGGS)₄-IFNα 2b(A145G)-Fc2 | 2 | DVVMTQSPLSLPVTPGEPASISCRSSQSLVHSNRNTYLHWYLQKPGQSP QLLIYKVSNRFSGVPDRFSGSGSGTDFTLKISRVEAEDVGVYYCSQNTH VPPTFGQGTKLEIKRTVAAPSVFIFPPSDEQLKSGTASVVCLLNNFYPRE AKVQWKVDNALQSGNSQESVTEQDSKDSTYSLSSTLTLSKADYEKHK VYACEVTHQGLSSPVTKSFNRGECGGGGSGGGGSGGGGSGGGGSCDL PQTHSLGSRRTLMLLAQMRRISLFSCLKDRHDFGFPQEEFGNQFQKAET IPVLHEMIQQIFNLFSTKDSSAAWDETLLDKFYTELYQQLNDLEACVIQ GVGVTETPLMKEDSILAVRKYFQRITLYLKEKKYSPCAWEVVRGEIMRS FSLSTNLQESLRSKEDKTHTCPPCPAPELLGGPSVFLFPPKPKDTLMISRT PEVTCVVVDVSHEDPEVKFNWYVDGVEVHNAKTKPREEQYNSTYRV VSVLTVLHQDWLNGKEYKCKVSNKALPAPIEKTISKAKGQPREPQVYT LPPSRDELTKNQVSLWCLVKGFYPSDIAVEWESNGQPENNYKTTPPVLD SDGSFFLYSKLTVDKSRWQQGNVFSCSVMHEALHNHYTQKSLSLSPGK |
| IFN-α 2b (comprising a natural signal peptide) | 3 | MALTFALLVALLVLSCKSSCSVGCDLPQTHSLGSRRTLMLLAQMRRISL FSCLKDRHDFGFPQEEFGNQFQKAETIPVLHEMIQQIFNLFSTKDSSAA WDETLLDKFYTELYQQLNDLEACVIQGVGVTETPLMKEDSILAVRKYF QRITLYLKEKKYSPCAWEVVRAEIMRSFSLSTNLQESLRSKE |
| IFN-α 2b (not comprising a natural signal peptide) | 4 | |
| IFN-α 2b-Fc1 | 5 | CDLPQTHSLGSRRTLMLLAQMRRISLFSCLKDRHDFGFPQEEFGNQFQK AETIPVLHEMIQQIFNLFSTKDSSAAWDETLLDKFYTELYQQLNDLEAC VIQGVGVTETPLMKEDSILAVRKYFQRITLYLKEKKYSPCAWEVVRAEI MRSFSLSTNLQESLRSKEDKTHTCPPCPAPELLGGPSVFLFPPKPKDTLM ISRTPEVTCVVVDVSHEDPEVKFNWYVDGVEVHNAKTKPREEQYNST YRVVSVLTVLHQDWLNGKEYKCKVSNKALPAPIEKTISKAKGQPREPQ VYTLPPSRDELTKNQVSLSCAVKGFYPSDIAVEWESNGQPENNYKTTPP VLDSDGSFFLVSKLTVDKSRWQQGNVFSCSVMHEALHNHYTQKSLSLS PGK |
| GC33(H)-Fc | 6 | QVQLVQSGAEVKKPGASVKVSCKASGYTFTDYEMHWVRQAPGQGLE WMGALDPKTGDTAYSQKFKGRVTLTADKSTSTAYMELSSLTSEDTAVY YCTRFYSYTYWGQGTLVTVSSASTKGPSVFPLAPSSKSTSGGTAALGCL VKDYFPEPVTVSWNSGALTSGVHTFPAVLQSSGLYSLSSVVTVPSSSLGT QTYICNVNHKPSNTKVDKKVEPKSCDKTHTCPPCPAPELLGGPSVFLFP PKPKDTLMISRTPEVTCVVVDVSHEDPEVKFNWYVDGVEVHNAKTKP REEQYNSTYRVVSVLTVLHQDWLNGKEYKCKVSNKALPAPIEKTISKA KGQPREPQVYTLPPSREEMTKNQVSLTCLVKGFYPSDIAVEWESNGQPE NNYKTTPPVLDSDGSFFLYSKLTVDKSRWQQGNVFSCSVMHEALHNH YTQKSLSLSPGK |
| GC33(L) | 7 | DVVMTQSPLSLPVTPGEPASISCRSSQSLVHSNRNTYLHWYLQKPGQSP QLLIYKVSNRFSGVPDRFSGSGSGTDFTLKISRVEAEDVGVYYCSQNTH VPPTFGQGTKLEIKRTVAAPSVFIFPPSDEQLKSGTASVVCLLNNFYPRE AKVQWKVDNALQSGNSQESVTEQDSKDSTYSLSSTLTLSKADYEKHK VYACEVTHQGLSSPVTKSFNRGEC |
| signal peptide of A chain and B chain of the present invention | 8 | MRAWIFFLLCLAGRALA |

### Example 2 construction, expression and purification of the plasmid for the recombinant protein

### 1. Plasmid construction, cell transfection and protein expression

The recombinant protein expression plasmid of the present invention was synthesized by GENEWIZ Company after codon optimization according to the protein sequence, ligated into pCDNA3.1 plasmid, and the sequence was verified by sequencing.

After filtering the obtained expression plasmids with a 0.22 µm filter, 50 µg plasmids were pipetted and added (the mass ratio of A chain and B chain expression plasmids was 2:1 or 3:1) into 2mL OptiPRO SFM Medium (GIBCO) and were mixed well. 160 µL transfection reagent ExpiFectamine CHO Reagent was pipetted and added into 2 mL OptiPRO SFM Medium and was mixed well. The obtained transfection reagent mixture solution was added to the mixture solution containing the plasmids and was mixed well. The mixture of the plasmids and the transfection reagent was slowly and evenly added to the host cell ExpiCHO-S (Thermo Fisher) suspension with a volume of 50 mL and a cell density of 6×10⁶ viable cells/mL, and the suspension was placed it in a 37°C, 8% CO2 incubator for culture. On day 1 (after 18 to 22 hours), 300 µL ExpiCHO Enhancer and 8 mL ExpiCHO Feed were added, and the culture temperature was lowered to 32°C. On day 5, the second feed was carried out, and 8 mL ExpiCHO Feed was supplemented, and the cell suspension was harvested after 12 days of culture. The cell suspension was centrifuged at 8000 rpm for 15 minutes. The supernatant obtained by centrifugation, that is, the cell culture harvest liquid was used for purification of the target protein.

### 2. Protein purification

### 2.1. Sample capture

The target protein was captured with Mabselect Sure (i.e., protein A, purchased from Cytiva) affinity filler from the recombinant protein expression supernatant of the present invention. The experimental process was as follows:
a): Balance: Balancing solution (50mM Tris-HCl, 150mM NaCl, pH7.2) was used to balance the chromatography column until the UV detection line was stable;
b) Sample loading: A sample pump was used to load the sample, with a retention time of 5 minutes and a loading capacity of ≤30 mg/ml;
c) Rebalance: Balancing solution (50mM Tris-HCl, 150mM NaCl, pH7.2) was used to wash the chromatography column for 5 column volumes;
d) Elution: Eluent (50mM NaAC-HAC, Ph3.5) was used to elute the target protein, and 1M Tris buffer was used to adjust the pH of the eluate to 5.5. The protein purity was detected by SDS-PAGE.

### 2.2. Sample purification

Aggregates and other impurities in the sample were removed from the recombinant protein TTM101-LC03-M3 expression supernatant of the present invention with SULFATE 650F filler (TOSOH). The experimental process was as follows:
a) Balance: Balancing solution (50mM NaAC-HAC, pH5.5) was used to balance the chromatography column until the UV detection line is stable;
b) Sample loading: A sample pump was used to load the sample, with a retention time of 5 minutes and a loading capacity of ≤50 mg/ml;
c) Rebalance: Balancing solution (50mM NaAC-HAC, pH5.5) was used to wash the chromatography column for 5 column volumes;
d) Elution: Eluent (50mM NaAC-HAC, 250Mm, pH5.5) was used to elute the target protein. The protein purity was detected by SDS-PAGE.

The purification method of the control sample Codrituzumab in Table 2 was same as 2.1.

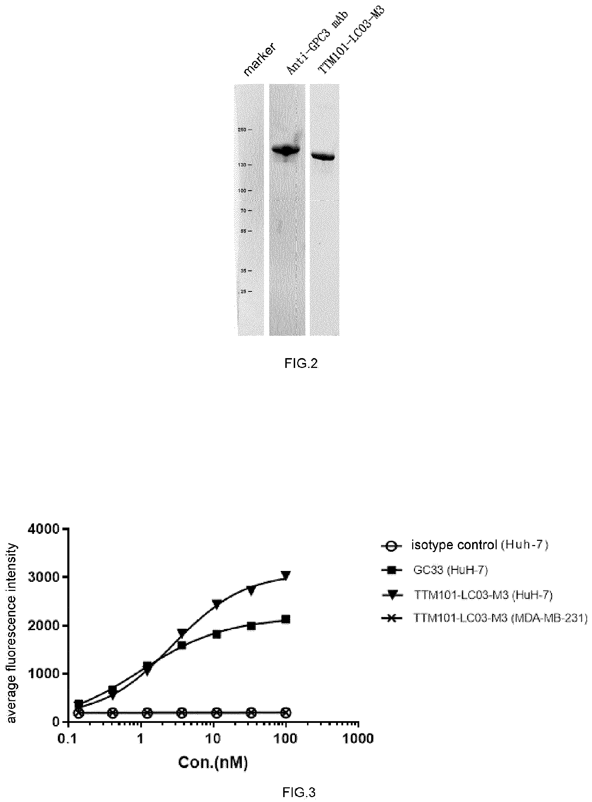

The theoretical molecular weight of the recombinant protein of the present invention shown in Table 1 was about 120kD, and the molecular weight of the control antibody Codrituzumab shown in Table 2 was 150KD. The results of non-reducing SDS-PAGE protein electrophoresis detection of the recombinant protein of the present invention (see Figure 1) after purification are shown in Figure 2. The results of non-reducing SDS-PAGE protein electrophoresis detection after purification (that is, after purification by SULFATE 650F) show that most impurities can be removed by cation exchange chromatography filler.

### Example 3 Detection of the binding activity of the recombinant protein of the present invention to GPC3-positive liver cancer cell line (double-positive expression cells, target cells) and GPC3-negative cell line (non-target cells)

The binding affinity of the recombinant protein of the present invention to the target GPC3-positive liver cancer cell line or GPC3-negative cell line was measured by flow cytometry.

The tested cells were GPC3-positive tumor cell line HuH-7 (Cell Bank of the Chinese Academy of Sciences in Shanghai) and GPC3-negative tumor cell line MDA-MB-231 (purchased from Nanjing Cobioer Biotechnology Co., Ltd.). Cells that grew well were collected and counted, centrifuged and were re-suspended with FACS buffer (PBS+2% FBS) to a concentration of 1×10⁶ cells/ml. Cells were added to a 96-well U-shaped plate (Cat. No.: 3799, Corning) at 100 µl/well, and 7 dilutions of TTM101-LC03-M3, GC33, and isotype control (starting from 100 nM, 3-fold gradient dilution, a total of 7 concentrations) were added. The plate was incubated at 4°C for 1 hour. After washing with FACS buffer, goat anti-human IgG (Alexa Fluor488 goat anti-human IgG (H+L), Invitrogen) was added, and the plate was incubated at 4°C for 1 hour. After washing with FACS buffer and re-suspension, the fluorescence value was detected by flow cytometry (Attune Nxt, invitrogen).

The experimental results are shown in Figure 3. Both TTM101-LC03-M3 and the anti-GPC3 monoclonal antibody GC33 have certain binding activity to the target cell HuH-7 cell, which expresses both GPC3 and IFNα receptors. Compared to the anti-GPC3 monoclonal antibody (anti-GPC3 mAb, GC33, i.e. the control sample Codrituzumab), the binding of TTM101-LC03-M3 to HuH-7 cells has a higher maximum average fluorescence intensity. At the same time, as shown in Figure 3, TTM101-LC03-M3 does not bind to the GPC3-negative (i.e., not expressing GPC3 but expressing IFNα receptor) non-target cell MDA-MB-231.

### Example 4 Detection of antibody-dependent cell-mediated cytotoxicity (ADCC) activity of the recombinant protein of the present invention

The ADCC activity of the recombinant protein of the present invention on the target GPC3-positive liver cancer cell line was measured through a reporter gene method.

The specific method was as follows:
1. Preparation of target cells and effector cells: Target cells HuH-7 (5E4/ml, 100µL/well) and Jurkat-NFAT-Luc-CD16A effector cells (2e6/ml, 50µL/well) were added to a 3904 plate.
2. The sample (50nM, 6X) was diluted to 8 concentration points, and was incubated at 50µL/well with target cells and effector cells at 37°C for 5-6 hours.
3. The cell plate was centrifuged at room temperature for 5 minutes, and 20 µL of the culture supernatant was pipetted and transferred into a 96-well plate (white transparent bottom).
4. QUANTI-Blue sloution detection reagent (Invivogen) was added at 50 µL/well, mixed well, and the signal value was detected with a microplate reader (SpectraMax M2).

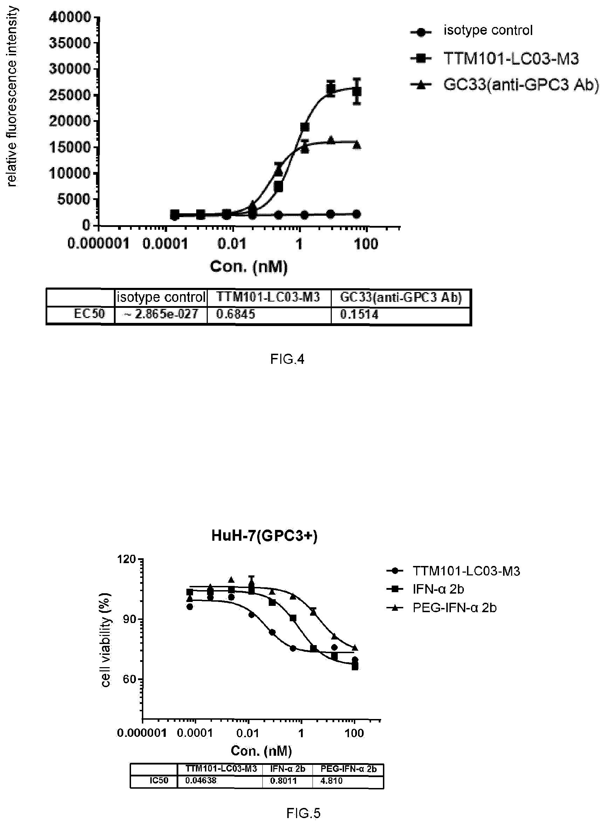

As shown in Figure 4, TTM101-LC03-M3 retained ADCC activity, and the ADCC activity of TTM101-LC03-M3 was comparable to that of anti-GPC3 monoclonal antibody (anti-GPC3 mAb, GC33, i.e., the control sample Codrituzumab).

### Example 5 Detection of proliferation inhibitory activity of the recombinant protein of the present invention

The proliferation inhibitory activity of the recombinant protein of the present invention on different tumor cell lines was measured by cell titer glo kit (Promega, Cat: G7558).

GPC3-positive cell line HuH-7 or GPC3-negative tumor cell line U266 (purchased from Nanjing Cobioer Biotechnology Co., Ltd.) or GPC3-negative tumor cell line MDA-MB-231(human breast cancer cells) (purchased from Nanjing Cobioer Biotechnology Co., Ltd.) were plated in a 96-well black-bottom transparent plate (Corning, 3904), and TTM101-LC01-M3 or control sample IFN-α2b (purchased from Nanjing GenScript Biotechnology Co., Ltd.), PEG-IFN-α 2b (purchased from Xiamen Amoytop Bioengineering Co., Ltd.) and anti-GPC3 antibody GC33 (starting from 100nM, 6-fold gradient dilution, a total of 9 points) were added. The plate was placed in a CO2 incubator at 37°C and was cultured for 3 days, and then was placed into Cell titer glo, Multimode Plate Reader (PerkinElmer, Envision2105) to detect the signal value.

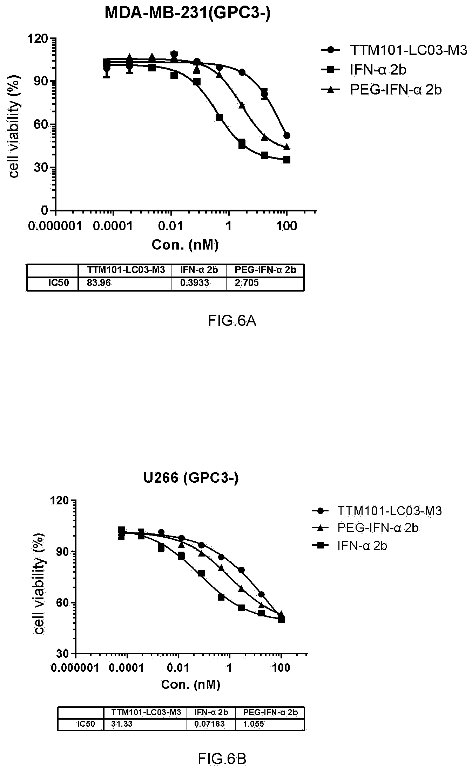

The results showed that the inhibitory activity (IC50) of TTM101-LC03-M3 on the proliferation of GPC3-positive (GPC3+) target cell HuH-7 was higher than those of the control samples IFN-α2b and PEG-IFN-α2b (see Figure 5), and was also higher than that of anti-GPC3 monoclonal antibody (anti-GPC3 mAb, GC33, i.e., the control sample Codrituzumab which has no inhibitory effect on HuH-7 proliferation). Specifically, as shown in Figure 5, the proliferation inhibitory activity of TTM101-LC03-M3 on the GPC3-positive (GPC3+) target cell HuH-7 (IC50=0.04638nM) was about 100 times stronger than that of the control sample PEG-IFN-α 2b (IC50=4.81nM). The proliferation inhibitory activity of TTM101-LC03-M3 on GPC3-negative non-target cells was significantly lower than those of the control samples IFN-α 2b and PEG-IFN-α 2b. Specifically, as shown in Figure 6A, the proliferation inhibitory activity of TTM101-LC03-M3 on the GPC3-negative (GPC3-) non-target cell MDA-MB-231 (IC50=83.96nM) was about 200 times weaker than that of the control sample IFN-α 2b (IC50=0.3933nM). As shown in Figure 6B, the proliferation inhibitory activity of TTM101-LC03-M3 on the GPC3-negative (GPC3-) non-target target cell U266 (IC50=31.33nM) was about 400 times weaker than that of the control sample IFN-α 2b (IC50=0.07183nM).

The above-mentioned results of the proliferation inhibitory activity of the recombinant protein of the present invention on GPC3-positive or GPC3-negative cells show that the recombinant protein TTM101-LC03-M3 of the present invention can significantly enhance the proliferation inhibitory activity of the IFN-α 2b functional fragment in TTM101-LC03-M3 by binding to GPC3 on GPC3-positive target cells. Unexpectedly, the proliferation inhibitory activity of the IFN-α 2b functional fragment in the recombinant protein TTM101-LC03-M3 of the present invention on non-target cells that do not express GPC3 was reduced by 200 times or even more than 400 times. The recombinant protein of the present invention only has a stronger proliferation inhibitory effect on target cells that have GPC3, but has a weaker effect on non-target cells that do not have GPC3. This shows that the recombinant protein of the present invention is extremely safe.

### Example 6 Detection of the proliferation inhibitory activity of the potential risk impurity of the recombinant protein of the present invention on non-target cells

In order to reduce the safety risk of potential impurities in the future preparation process of the recombinant protein, the present invention compared the potential risk impurity, B chain homodimer of TTM101-LC03-M3 and its inhibition of the proliferation of non-target target cells.

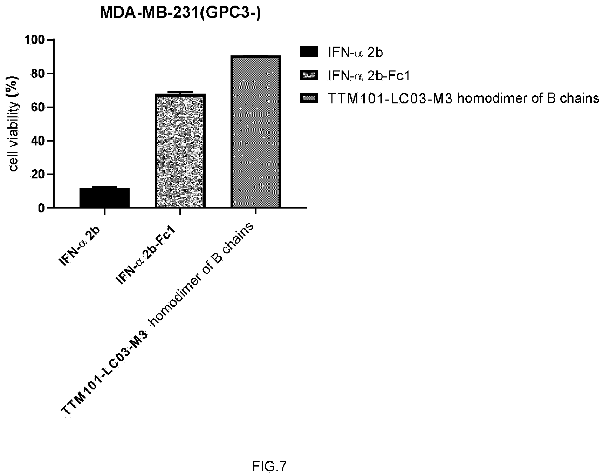

In the purification process of TTM101-LC03-M3 (as described in Example 2), the B chain homodimer of TTM101-LC03-M3 (homodimer having a molecular weight of about 140kD) was isolated, and its proliferation inhibitory activity and that of IFN-α 2b-Fc1 (the right arm homodimer of the recombinant protein structure shown in Figure 3 of CN108864290B) on non-target cell (GPC3 negative cell) MDA-MB-231 was detected. The results are shown in Figure 7. At a concentration of 3nM, IFN-α2b inhibited the proliferation of MDA-MB-231 (GPC3 negative cell, non-target cell) by 88.3%, and IFN-α2b-Fc1 inhibited the proliferation of MDA-MB-231 (GPC3 negative cell, non-target cell) by 32.1% (approximately 56.2% lower than that of IFN-α2b). The B chain homodimer of TTM101-LC03-M3 inhibited the proliferation of MDA-MB-231 (GPC3 negative cell, non-target cell) by only 9.3% (approximately 79% lower than IFN-α 2b, and approximately 22.8% lower than IFN-α 2b-Fc1).

In summary, potential risk impurities only had a very weak effect on non-target cells. That is, the potential safety risks or potential toxic side effects were extremely low.

### Example 7 Detection of in vivo anti-tumor activity of the recombinant protein of the present invention

Anti-tumor efficacy of GPC-targeting recombinant protein in BALB/c nude mouse HuH-7 subcutaneous tumor model

A Huh-7 tumor mass of ~30 mm³ was inoculated into the back of right necks of BALB/c nude mice. At the same time as the inoculation, the experimental animals were marked with ear tags as the only confirmation mark for subsequent experiments. When the tumor grew to the average tumor volume of 150mm³, drug administration began in random groups, with 6 mice in each group. All mice were administered by intravenous injection, and the administration volume was 10 mL/kg. The administration was continued for 3 weeks, twice a week, for a total of 6 administrations.

The experimental indicator was to examine whether tumor growth was inhibited, delayed or cured. Tumor diameter was measured twice weekly using a vernier caliper. The calculation formula of tumor volume was: V=0.5a×b², wherein a and b represented the long and short diameters of the tumor respectively. Statistical analysis included mean and standard error (SEM) of tumor volume at each time point for each group. Comparisons between two groups were analyzed using one-tailed T test, and GraphPad Prism was used for all data analysis. p<0.05 was deemed to be a significant difference.

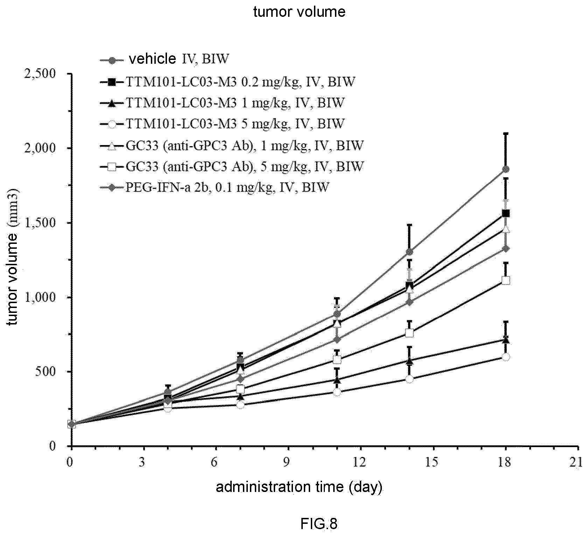

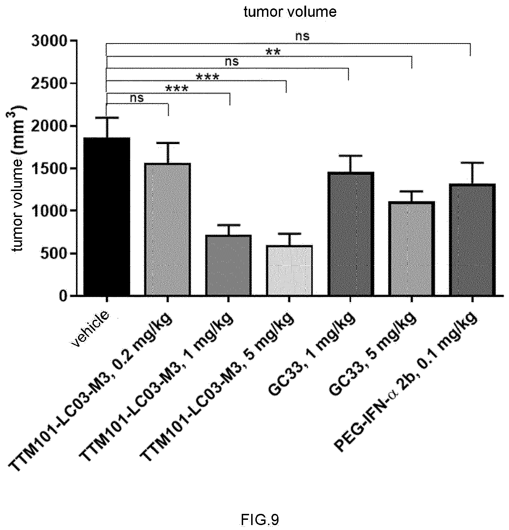

The results are shown in Figures 8 and 9 and Table 4. TTM101-LC03-M3 inhibited tumor growth in a dose-dependent manner, and the anti-tumor effect was better than that of GC33 at the same dosage, and was also better than that of high-dosage of PEG-IFN-α2b (at a dosage higher than clinically tolerance dose). The above results indicate that TTM101-LC03-M3 can effectively inhibit the tumor growth of liver cancer cells.

**Table 4. Evaluation of the anti-tumor efficacy of TTM101-LC03-M3 on the subcutaneously transplanted female BALB/c nude mouse model of Huh-7 tumor (calculated based on the tumor volume on day 18 after administration)**

| group | Treatment by the compound | Volume of tumor (mm³)^{a} | T/C (%) | TGI (%) | *P* value^{b} |
|---|---|---|---|---|---|
| | | | | | (vs vehicle) |
| 1 | vehicle, IV, BIW | 1860±237 | - | - | - |
| 2 | TTM101-LC03-M3, 0.2 mg/kg,IV, BIW | 1563±235 | 84.0 | 17.4 | *p*>0.05 |
| 3 | TTM101-LC03-M3, 1 mg/kg,IV, BIW | 719±116 | 38.6 | 66.7 | *p*<0.001 |
| 4 | TTM101-LC03-M3, 5 mg/kg IV, BIW | 597±136 | 32.1 | 73.8 | *p*<0.001 |
| 5 | GC33, 1 mg/kg,IV, BIW | 1461±186 | 78.5 | 23.3 | *p*>0.05 |
| 6 | GC33, 5 mg/kg,IV, BIW | 1112±118 | 59.8 | 43.8 | *p*<0.01 |
| 7 | PEG-IFN-α 2b, 0.1 mg/kg,IV, BIW | 1325±242 | 71.2 | 31.3 | *p*>0.05 |

| | | | | | |
|---|---|---|---|---|---|
| Note: a. mean ± SEM b. Calculation of p value: Each administration group was compared to Vehicle and analyzed using one-tailed T test. | | | | | |

### Example 8 Investigation of freeze-thaw stability of the recombinant protein

The existing recombinant human albumin interferon-α 2b fusion protein has poor freeze-thaw stability and is not suitable for repeated freezing and thawing. For example, PEGylated long-acting interferon cannot be frozen and shaken, and requires stricter transportation and storage conditions. In order to study the freeze-thaw stability of the recombinant protein of the present invention, the recombinant protein of the present invention was subjected to repeated freeze-thaw stability tests. The protein was placed in 20mM NaAc (PH=5) buffer, and frozen and thawed five times at -40°C. Samples before and after freezing and thawing were analyzed for purity (size exclusion chromatography, SEC) and appearance. The results are shown in Table 5. TTM101-LC03-M3 had good freeze-thaw stability, with purity above 97%. The appearance was clear after 5 repeated freeze-thaws, indicating that the freeze-thaw stability of the recombinant protein of the present invention was significantly better than that of IFN-α2b monomer or PEGylated IFN-α2b.

**Table 5 Freeze-thaw stability of the recombinant protein of the present invention**

| No. | SEC-HPLC (%) | No freeze-thaw | | | 5 times of freeze-thaw | | |
|---|---|---|---|---|---|---|---|
| | | Monomer | multimer | Impurity of low molecular weight | Monomer | multimer | Impurity of low molecular weight |
| 1 | TTM101-LC03-M3 | 99.11 | 0.89 | ND | 97.68 | 2.32 | ND |

The use of any and all examples, or exemplary language (e.g, "such as") provided herein is intended merely to better illuminate the invention and does not pose a limitation on the scope of the invention unless otherwise claimed. No language in the specification should be construed as indicating any non-claimed element as essential to the practice of the invention.

All publications cited in this specification are herein incorporated by reference to the same extent as if each individual publication was specifically and individually indicated to be incorporated by reference. Furthermore, any theory, mechanism, demonstration or discovery described herein is intended to further enhance the understanding of the invention and is not intended to limit the invention in any way to such theory, mechanism, demonstration or discovery. While the invention has been shown and described in detail in the drawings and foregoing description, the invention is to be regarded as illustrative rather than restrictive.

## Claims

1. An antibody-interferon α fusion protein targeting GPC3, **characterized in that** the fusion protein consists of an A chain and a B chain; wherein the amino acid sequence of the A chain is as shown in SEQ ID NO: 1, and the amino acid sequence of the B chain is as shown in SEQ ID NO:2.

2. An isolated nucleic acid, **characterized in that** the nucleic acid encodes the antibody-interferon α fusion protein according to claim 1.

3. A recombinant expression vector, **characterized in that** the recombinant expression vector comprises the nucleic acid according to claim 2.

4. A transformant, **characterized in that** the transformant comprises the recombinant expression vector according to claim 3.

5. A method for preparing an antibody-interferon α fusion protein targeting GPC3, **characterized in that** the method comprises the following steps: culturing the transformant according to claim 4, and obtaining the antibody-interferon α fusion protein from the culture.

6. A pharmaceutical composition, **characterized in that** the pharmaceutical composition comprises the antibody-interferon α fusion protein according to claim 1 and a pharmaceutically acceptable carrier.

7. Use of the antibody-interferon α fusion protein according to claim 1 or the pharmaceutical composition according to claim 6 in the manufacture of a medicament for the treatment, auxiliary treatment or prevention of a disease.

8. The use according to claim 7, **characterized in that** the disease is a GPC3-positive related disease.

9. The use according to claim 8, **characterized in that** the GPC3-positive related disease is selected from a tumor, a liver disease and a viral infection disease.

10. The use according to claim 9, **characterized in that** the tumor is selected from breast cancer, intestinal cancer, pancreatic cancer, esophageal cancer, ovarian cancer, gastric cancer, prostate cancer, kidney cancer, cervical cancer, myeloma, lymphoma, leukemia, thyroid cancer, uterine cancer, bladder cancer, neuroendocrine cancer, head and neck cancer, liver cancer, nasopharyngeal cancer, testicular cancer, lung cancer, melanoma, skin cancer, sarcoma, glioma, mesothelioma and myelodysplastic syndrome.

11. The use according to claim 10, **characterized in that** the intestinal cancer comprises colorectal cancer, the ovarian cancer comprises yolk sac tumor, the neuroendocrine cancer comprises Merkel cell carcinoma, the lung cancer comprises small cell lung cancer and non-small cell lung cancer, the skin cancer comprises basal cell skin cancer and squamous cell skin cancer, the sarcoma comprises dermatofibrosarcoma protuberans, the gliomas comprises glioblastoma, and the uterine cancer comprises endometrial cancer and uterine sarcoma.
